# EUROPEAN PATENT APPLICATION

(11) **EP 1 897 528 A1**
(43) Date of publication of application: **12.03.2008**
(21) Application number: 07115667.3
(22) Date of filing: 04.09.2007
(51) Int. Cl.: A61K 8/19, A61Q 19/10

(54) **Skin-care water**

(30) Priority: 04.09.2006 JP 2006238580; 26.04.2007 JP 2007116385
(71) Applicant: MIURA CO., LTD., Matsuyama-shi Ehime 799-2696 (JP)
(72) Inventor: Yoshinari, Yuji, Matsuyama-shi Ehime 799-2696 (JP); Takai, Masaki, Matsuyama-shi Ehime 799-2696 (JP); Hatori, Makoto, Matsuyama-shi Ehime 799-2696 (JP)
(74) Representative: Marchant, James Ian

(57) **Abstract**

The skin-care water, which is able to safely improve skin health conditions, such as dry skin, contains water from which polyvalent cations are removed and to which sodium ions are added, and may contain fragrant materials according to need. When the skin-care water is applied to skin, for example, by means of taking a bath, it improves moisture retention of skin, moisture content of skin, elasticity, texture density and the like without providing side effects, as is often not the case when a skin-care water containing chemical substances is used. Accordingly, the skin-care water is useful as a moisturizing agent for skin, and an improving agent for dry skin, itchy feeling, skin scale and atopic dermatitis.

## Description

### Technical Field

The present invention relates to a skin-care water for improving skin health condition.

### Background Art

Human skin is susceptible to such influences as friction with clothes, particularly those using chemical fibers, direct sunlight, acid rain, jobs using water, dissolution of sebum in taking a bath, artificially introduced drying environment by air conditioners and the like, and the health condition thereof can be easily damaged. Specifically, rough skin and dry skin are occurring, and if the symptom progresses, itchy feeling or scale can be developed in some cases. Particularly, skin is apt to lose moisture retention owing to aging and to be dry, and thus itchy feeling and a scale symptom tend to progress more. In addition, there are many cases that skin health condition is damaged by onset of dermatitis such as atopic dermatitis. Accordingly, various skin-care agents have been proposed in order to improve functions of skin whose health condition has been damaged.

For example, a skin-care agent using borneol (Japanese Unexamined Patent Publication (Kokai) No. 6-211713 (JP 1994-211713 A)), that using a culture medium of actionmyces (Japanese Unexamined Patent Publication (Kokai) No. 5-25053 (JP 1993-25053 A)), that using oil and fat such as docosahexanoic acid (DHA) or linolenic acid (Japanese Unexamined Patent Publication (Kokai) No. 2-290812 (JP 1990-290812 A)) and that using an extract from acerola seed (Japanese Unexamined Patent Publication (Kokai) No. 2006-117542 (JP 2006-117542 A)) have been proposed.

However, any of the above various skin-care agents contain chemical substances as active constituents, even though they are natural products-oriented. Therefore, there are possibilities that the skin-care agents have risks of providing some side effects onto human body, and that the side effects can be remarkable depending on one's constitution.

An object of the present invention is to improve skin health condition safely.

The term "skin" is the concept including mucous and cornea in the present application.

### Summary of the Invention

A skin-care water of the present invention contains water, from which polyvalent cations are removed and to which sodium ions are added. When the skin-care water is applied to skin, skin moisture increases by the action thereof without a side effect, and the skin health condition improves. Accordingly, the skin-care water can be used as, for example, a moisturizing agent for skin and an improving agent for dry skin, itchy feeling, skin scale and atopic dermatitis.

Other objects and effects of the present invention will be described in detail hereinafter.

### Brief Description of the Drawings

Fig. 1 is a graph showing the results of stratum corneum moisture content measured in Evaluation 1 of Example A.
Fig. 2 is a graph showing the results of skin elasticity measured in Evaluation 1 of Example A.
Fig. 3 is a graph showing the results of texture density measured in Evaluation 1 of Example A.
Fig. 4 is a graph showing the results of clinical dermatitis score measured in Example C.
Fig. 5 is a graph showing the results of a number of scratching measured in Example C.
Fig. 6 is a graph showing the results of a total scratching time measured in Example C.
Fig. 7 is a graph showing the results of a transepidermal water loss measured in Example C.

### Description of the Preferred Embodiment

The skin-care water of the present invention contains water from which polyvalent cations are removed and to which sodium ions are added (hereafter such water is called "functional water" in some cases). The functional water is obtained by treatment of water (raw water), such as tap, ground, river, lake and well water, with a cation exchange resin. In this treatment, a calcium ion (bivalent cation), magnesium ion (bivalent cation), copper ion (bivalent cation), iron ion (bivalent and trivalent cations), aluminum ion (trivalent cation) and the like contained in the raw water are exchanged with a sodium ion (monovalent cation) contained in the cation exchange resin.

The cation exchange resin used for the treatment of raw water is a synthetic resin, wherein a sulfonic acid group is introduced to a matrix of a cross-linked three-dimensional polymer such as a copolymer of styrene and divinylbenzene, and the sulfonic acid group form a sodium salt.

In the functional water, it is preferable that a concentration of polyvalent cations is commonly adjusted to less than 0.2 mmol/l, and particularly preferable to be adjusted to less than the measurement limit, which signifies substantially zero level. Here, the concentration of polyvalent cations denotes a concentration measured on the basis of ICP emission spectroscopic analysis.

While, in the functional water, it is preferable that a concentration of a sodium ion is commonly adjusted to 0.3 mmol/l or more and less than 500 mmol/l, and more preferable to be adjusted to 0.5 mmol/l or more and less than 200 mmol/l. Here, the concentration of a sodium ion denotes a concentration measured on the basis of ICP emission spectroscopic analysis.

The skin-care water of the present invention may contain some other components other than the above functional water to the extent that they do not spoil the objects of the present invention. Examples of other components include fragrant materials such as grapefruit oil, spearmint oil, nutmeg oil and mandarin oil, and antioxidants such as tocopherol, ascorbyl stearate ester, sodium erythorbate, ascorbic acid, citric acid and dibutyl hydroxytoluene. Two or more of the fragrant materials and antioxidants may be used in combination.

The skin-care water of the present invention can be prepared by treating the raw water with the above cation exchange resin, and then properly adding the above other components to the resulting functional water according to need.

Skin to which the skin-care water of the present invention can be applied includes entire body skin including mucous and cornea, and is not particularly limited. Although an application method to the skin is not particularly limited, it is preferable to employ a method that enables the skin to be fully moistened with the skin-care water, such as taking a bath, taking a shower, face wash, hand wash and spraying therewith. The skin to which the skin-care water was applied can be dried as it is, but is preferably wiped out to remove water.

Since the skin-care water of the present invention consists primarily of the above functional water, it can be used without minding one's constitution. In addition, the skin-care water can increase moisture in the skin and improve moisture content, elasticity (tightness) and texture density of skin without providing any side effect, as is often not the case when a skin-care agent containing chemical substances is used. The effects are remarkable when the skin-care water of the present invention is used for stratum cornea such as elbows, knees and heels, dry skin and the skin showing a symptom of atopic dermatitis. The skin-care water not only improves a scale symptom observed in dry skin, but also alleviates itchy feeling that is a major complaint made by those suffering from atopic dermatitis or serious dry skin. Accordingly, the skin-care water is useful as a moisturizing agent for skin, and an improving agent for dry skin, itchy feeling, scale and atopic dermatitis.

The above effects are likely to be achieved when the skin-care water of the present invention is continuously applied to skin, particularly applied over one week or longer.

In the above embodiment, water from which polyvalent cations are removed and to which sodium ions are added is used as functional water. However, the functional water may be such that polyvalent cations are removed and alkali metal ions other than sodium ions, such as potassium ions, are added. This functional water can be obtained by treating raw water using the above cation exchange resin, wherein sulfonic acid group forms an alkali metal salt such as a potassium salt.

### Examples

### [Example A]

An improvement state of skin was studied on 8 examinees who took a bath (full bath) everyday for 4 weeks using the skin-care water of the present invention. The examinees were female aging from 30 to 47 years old (average age: 36.9 years old), who were diagnosed as dry skin. The skin-care water used herein consists only of water obtained by treating tap water with a cation exchange resin, and satisfied the conditions that a concentration of polyvalent cation is less than 0.2 mmol/l and a concentration of sodium ions is 0.3 mmol/l or more and less than 500 mmol/l.

### Evaluation 1

Each item of stratum corneum moisture content, skin elasticity and texture density was measured on every examinee by the following methods. The mean value of each item was compared among the day when the test was started, 2 weeks after the test start and 4 weeks after the test start.

### (Stratum corneum moisture content)

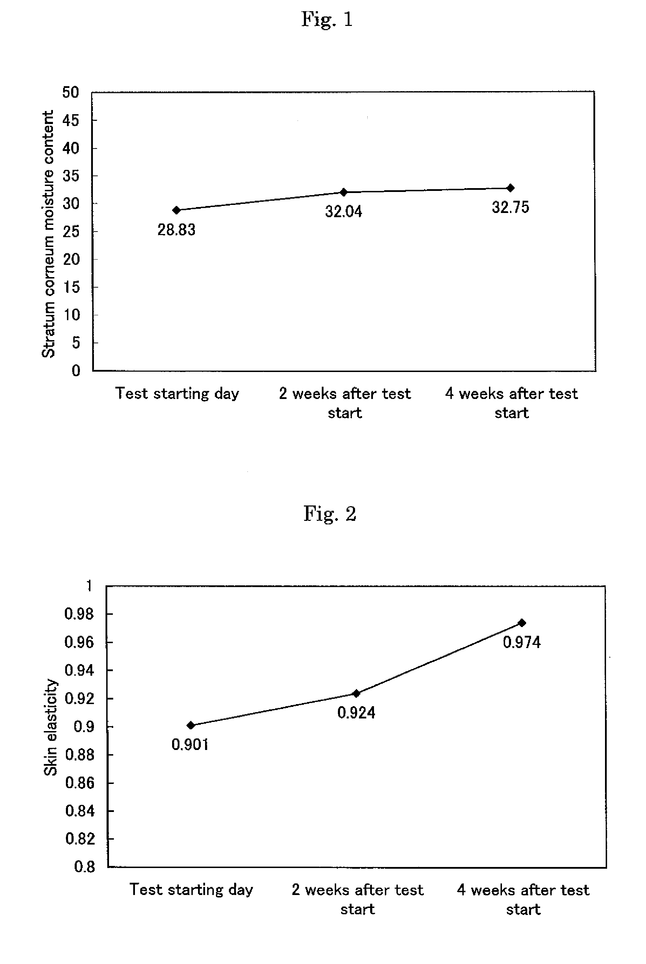

An electric characteristic of skin, that is, capacitance of skin surface was measured to determine stratum corneum moisture content with a measuring device of stratum corneum moisture content (trade name of "Corneometer CM825" manufactured by Courage + Khazaka Electronic GmbH). The measurement was conducted 3 times, and the mean value was served as the measurement value. The results are shown in Fig. 1.

### (Skin elasticity)

Skin electricity was measured using a measuring device of skin elasticity (trade name of "Cutometer SEM575" manufactured by Courage + Khazaka Electronic GmbH), which is equipped with a measurement probe having a suction hole, that is, a negative pressure aspirator, a pressure sensor, and a computer for operation thereof and data processing. In this measurement, when the probe is guided to a skin surface to start the measurement, a negative pressure is applied to the probe suction hole and thus the skin inside the suction hole is sucked in. Then, a height of the sucked skin was measured with a light sensor contactlessly without generation of friction or mechanical action. The measurement was conducted 4 times, and the mean value was served as the measurement value. The results are shown in Fig. 2.

### (Texture density)

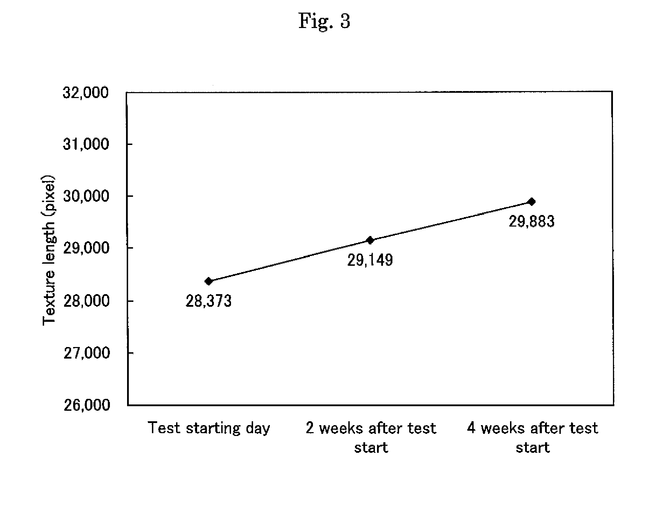

Texture density was measured by applying image analysis software (trade name of "Skin Analysis Software" manufactured by Inforward Inc.) for the purpose of evaluating a state of the texture distribution to a color image of skin obtained by a device with a trade name of "microscope VI-27" manufactured of FineOpt Co., Ltd. Specifically, image data processing was applied by the above image analysis software to a color image obtained so as to emphasize and extract the texture region, and a total length of the texture region (unit: pixel) was calculated. The results are shown in Fig. 3. Texture density denotes a percentage (%) of a texture length in an area of the obtained color image, and is therefore substantially in a relation of "texture length (pixel)" = "texture density (%)".

### Evaluation 2

On the same day when each item in Evaluation 1 was measured, drying condition of skin and existence or nonexistence of scale were evaluated on every examinee, according to a diagnosis by a doctor. The results are shown in Table 1. In Table 1, evaluation criteria on each item are as follows.

### (Drying condition)

None: The symptom is not observed.
Minor: The symptom is slightly observed.
Mild: The symptom is somewhat observed.
Moderate: The symptom is clearly observed.
Severe: The symptom is remarkably observed.

### (Existence or nonexistence of scale)

None: The symptom is not observed.
Minor: The symptom is slightly observed.
Mild: The symptom is somewhat observed.
Moderate: The symptom is clearly observed.
Severe: The symptom is remarkably observed.

**Table 1**

| | | None | Minor | Mild | Moderate | Severe |
|---|---|---|---|---|---|---|
| Drying condition | Test starting day | 0 | 1 | 5 | 2 | 0 |
| | Two weeks after test start | 0 | 5 | 3 | 0 | 0 |
| | Four weeks after test start | 0 | 7 | 1 | 0 | 0 |
| Existence or nonexistence of scale | Test starting day | 1 | 6 | 1 | 0 | 0 |
| | Two weeks after test start | 1 | 7 | 0 | 0 | 0 |
| | Four weeks after test start | 7 | 0 | 1 | 0 | 0 |

### Evaluation 3

On the same day when each item in Evaluation 1 was measured, itchy feeling of skin based on the examinees' own complaint was evaluated according to a diagnosis by a doctor. The results are shown in Table 2. In Table 2, evaluation criteria on itchy feeling are as follows.
None: The symptom is not observed.
Minor: The symptom is slightly observed.
Mild: The symptom is somewhat observed.
Moderate: The symptom is clearly observed.
Severe: The symptom is remarkably observed.

**Table 2**

| | | None | Minor | Mild | Moderate | Severe |
|---|---|---|---|---|---|---|
| Itchy feeling | Test starting day | 3 | 1 | 3 | 1 | 0 |
| | Two weeks after test start | 6 | 2 | 0 | 0 | 0 |
| | Four weeks after test start | 8 | 0 | 0 | 0 | 0 |

### Evaluation 4

The results of questionnaire relating to self-evaluation on the examinees' own skin are shown in Table 3, which was implemented to each examinee before and after the test.

**Table 3**

| | | Feel | Somewhat feel | Normal | Not quite feel | Not feel |
|---|---|---|---|---|---|---|
| There is a taut feeling after taking a bath | Before test start | 3 | 2 | - | 1 | 2 |
| | After test termination | 0 | 0 | - | 4 | 4 |
| Skin is dry | Before test start | 4 | 2 | 2 | 0 | 0 |
| | After test termination | 1 | 3 | 1 | 2 | 1 |
| Skin is smooth | Before test start | 0 | 3 | 2 | 1 | 2 |
| | After test termination | 2 | 4 | 1 | 0 | 1 |
| Skin has tightness and elasticity | Before test start | 0 | 0 | 5 | 1 | 2 |
| | After test termination | 0 | 3 | 4 | 0 | 1 |
| Skin texture is finer | Before test start | 0 | 2 | 4 | 1 | 1 |
| | After test termination | 0 | 5 | 2 | 1 | 0 |

### [Example B]

An improvement state of skin was studied on 18 examinees who took a bath (full bath) everyday for 4 weeks using the skin-care water of the present invention. The examinees were Japanese female aging from 21 to 39 years old (average age: 25.8 years old), who were diagnosed that severity of eruption caused by atopic dermatitis is mild or lighter in accordance with the standard stipulated in "Atopic Dermatitis Treatment Guideline: 2004 revised edition" compiled by Japan Dermatological Association.

The skin-care water used herein consisted only of water obtained by treating tap water with a cation exchange resin, which satisfied the conditions that a concentration of polyvalent cation is less than 0.2 mmol/l and a concentration of sodium ions is 0.3 mmol/l or more and less than 500 mmol/l. The examinees abided by the following items during the test period.
(1) Do not take new internal medicines nor use new external medicines (dosing or applying), although internal and external medicines that have been conventionally used may be continued to use.
(2) Do not change cosmetics to other products, although cosmetics (cleansing, lotion, milky lotion, foundation, body cream, shampoo, body soap and the like) that have been conventionally used may be continued to use.
(3) Do not get an excessive suntan.
(4) Do not shave hair on a region to be observed that will be described later.

### Evaluation 1

On the particular regions (skin of forearms and lower thigh), clinical examination and measurement of stratum corneum moisture content, transepidermal water loss and skin elasticity (forearms only) were conducted on every examinee on the day of the test start, 2 weeks after and 4 weeks after the test start by the following method.

### (Clinical examination)

According to observation and inquiry by a dermatologist, dry condition, development of scale, erythema and itchy feeling at the regions were evaluated.

### (Stratum corneum moisture content)

Stratum corneum moisture content was measured in the same manner as in Evaluation 1 of Example A.

### (Transepidermal water loss)

Transepidermal water loss was measured by using a measuring device of transepidermal water loss (trade name of "VAPO meter" manufactured by Keystone Electric Corp.). The measurement was conducted twice, and the mean value was served as a measurement value.

### (Skin elasticity)

Skin elasticity was measured in the same manner as in Evaluation 1 of Example A.

### <Results>

With regard to each item, the mean values of 18 examinees were compared from day to day during the test period. The results are shown in Table 4. The evaluation contents shown in Table 4 are as follows.
B: Improved
A: Improved more than the state of B
C: Unimproved
←: Maintaining the state of B, but unimproved furthermore

**Table 4**

| | Forearm | | Lower thigh | |
|---|---|---|---|---|
| | 2 weeks after | 4 weeks after | 2 weeks after | 4 weeks after |
| Dryness | B | A | B | A |
| Development of scale | B | A | B | A |
| Erythema | B | A | C | B |
| Itchy feeling | B | A | B | A |
| Stratum corneum moisture content | B | A | B | A |
| Transepidermal water loss | C | B | C | B |
| Skin elasticity | B | ← | - | - |

According to Table 4, the skin-care water of the present invention is considered to be useful for improvement of atopic dermatitis.

### Evaluation 2

All of the examinees were surveyed by questionnaire concerning esthesis of their own skin before the test start and 4 weeks after the test start. The results are shown in Table 5.

**Table 5**

| | | Feel | Somewhat feel | No change or Normal | Not quite feel | Not feel |
|---|---|---|---|---|---|---|
| There is a taut feeling after taking a bath | Before test start | 50 | 50 | 0 | 0 | 0 |
| | After test termination | 0 | 11 | 6 | 50 | 33 |
| Skin is softer | Before test start | 0 | 11 | 45 | 33 | 11 |
| | After test termination | 6 | 11 | 49 | 28 | 6 |
| Skin is dry | Before test start | 83 | 17 | 0 | 0 | 0 |
| | After test termination | 22 | 56 | 11 | 11 | 0 |
| Skin is smooth | Before test start | 0 | 17 | 17 | 38 | 28 |
| | After test termination | 6 | 49 | 17 | 17 | 11 |
| Skin is rough | Before test start | 56 | 33 | 11 | 0 | 0 |
| | After test termination | 17 | 22 | 50 | 0 | 11 |
| Skin has tightness and elasticity | Before test start | 6 | 0 | 28 | 44 | 22 |
| | After test termination | 6 | 33 | 33 | 17 | 11 |
| Skin texture is finer | Before test start | 6 | 0 | 39 | 33 | 22 |
| | After test termination | 6 | 28 | 32 | 17 | 17 |
| There is itchy feeling on skin during daytime | Before test start | 43 | 28 | 17 | 6 | 6 |
| | After test termination | 11 | 33 | 11 | 28 | 17 |
| There is itchy feeling on skin at night | Before test start | 39 | 33 | 6 | 11 | 11 |
| | After test termination | 11 | 22 | 17 | 28 | 22 |
| (Unit: %) | | | | | | |

### [Example C]

### Test 1

Back of 10 NC/Nga mice developing a symptom of atopic dermatitis (those with clinical dermatitis score of about 7 to about 8) was cleaned with the skin-care water of the present invention and a cleaning agent once a day over 3 weeks (except Saturday and Sunday). The skin-care water used herein consisted only of water obtained by treating tap water with a cation exchange resin, which satisfied the conditions that a concentration of polyvalent cations is less than 0.2 mmol/l and a concentration of sodium ions is 0.3 mmol/l or more and less than 500 mmol/l. The cleaning agent comprised a liquid soap (an aqueous solution of fatty acid potassium salt) manufactured by Miyoshi Oil & Fat Co., Ltd. diluted twice with the skin-care water.

The back of the mouse was cleaned as follows: cleaned with a cotton bud, which was moisturized with the skin-care water and then impregnated with the cleaning agent; cleaned with sanitary cotton impregnated with the skin-care water 3 times so as to remove the cleaning agent; and wiped out with dry cotton to remove water. There occurred no case of side effect symptom such as mouse's death during the test period and after the test termination.

### Test 2

Mice were cleaned in the same manner as in Test 1, except for changing the skin-care water and the cleaning agent to tap water supplied in Fuchu city, Tokyo Japan, and a liquid soap (an aqueous solution of fatty acid potassium salt) manufactured by Miyoshi Oil & Fat Co., Ltd. diluted twice with the tap water, respectively.

### Evaluation

With regard to respective groups of mice to which cleaning was implemented in Test 1 and Test 2, clinical dermatitis score, scratching behavior and transepidermal water loss were measured respectively, and the improvement state in the symptom of atopic dermatitis was studied. The measurement methods and results of each item are as follows. Each result includes a result given by mice, which are the similar 10 mice as those used in Test 1 and Test 2 and are treated with nothing.

### (Clinical dermatitis score)

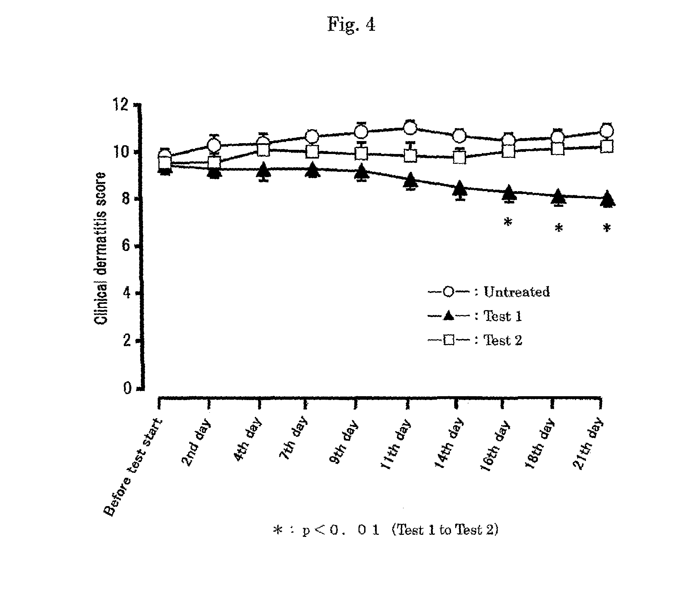

According to a method described in Immunogenetics 53: 15-21 (2001) "A major determinant quantitative-trait locus responsible for atopic dermatitis-like skin lesions in NC/Nga mice is located on Chromosome 9", the clinical dermatitis score was measured every Monday, Wednesday and Friday after the test start. The result is shown in Fig. 4. The result in Fig. 4 is shown as a mean value of 10 mice ± standard error. The significant difference test of Test 1 to Test 2 was conducted using a Dunnett-type multiple comparison test. The statistical significant difference was determined as significant when a risk rate p satisfied < 0.01. According to Fig. 4, a significant decline is recognized in the clinical dermatitis score on the 16^{th} day and after in Test 1, as compared with Test 2 and the untreated.

### (Scratching behavior)

Using a SCLABA system (trade name) manufactured by Noveltec Inc., a number that a mouse scratched on its back and the total scratching time during a predetermined time (20 min.) were measured before and after the test. The SCLABA system (trade name) marks a head and hind legs of a mouse with different colors of nonirritating fluorescent pigments, and films the behavior of the mouse with a digital video camera for a certain period of time to process the data. In the data processing, the state that a distance between the marked two colors came closer than a predetermined threshold value was regarded as a scratching behavior, and wrong recognition frames such as the mouse's rising behavior were deleted; and hereby, the system was specialized in the mouse's scratching behavior, and detection and quantification were materialized.

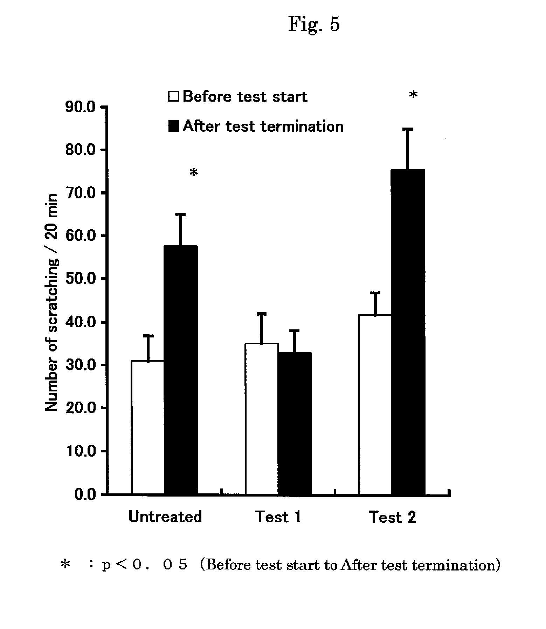

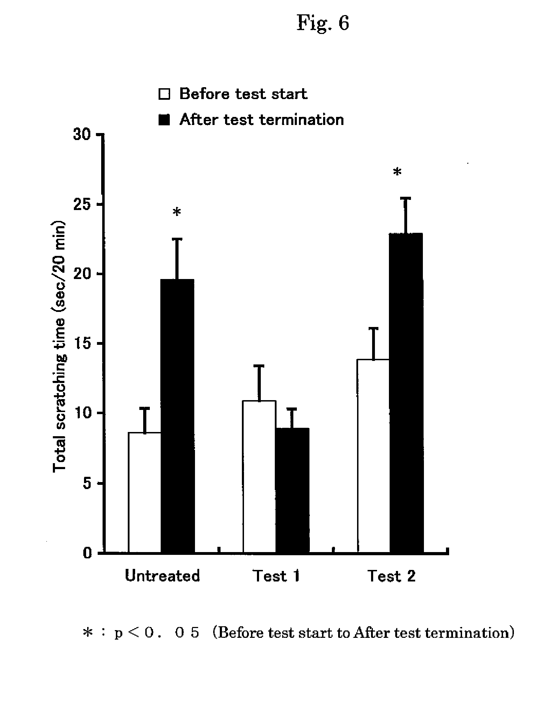

The results of the number of scratching and the total scratching time are shown in Fig. 5 and Fig. 6, respectively. The results of Fig. 5 and Fig. 6 are shown as a mean value of 10 mice ± standard error. The respective significant difference test of Test 2 and the untreated was conducted using a Dunnett-type multiple comparison test. The statistical significant difference was determined as significant when a risk rate p satisfied < 0.05. According to Fig. 5 and Fig. 6, it was learned that both the number of scratching and the total scratching time after the test termination decreased as compared with those before the test start in Test 1, while both the number of scratching and the total scratching time after the test termination significantly increased as compared with those before the test start in Test 2 and the untreated.

### (Transepidermal water loss)

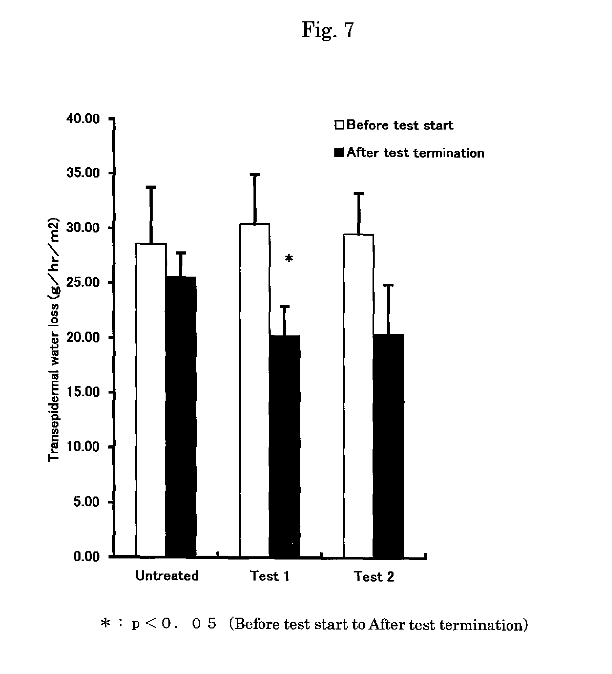

Using a transepedermal water loss measurement device, a transepidermal water loss was measured before and after the test. The result is shown in Fig. 7. The result of Fig. 7 is shown as a mean value of 10 mice ± standard error. The significant difference test of Test 1 before and after the test was conducted using a Dunnett-type multiple comparison test. The statistical significant difference was determined as significant when a risk rate p satisfied < 0.05. According to Fig. 7, a significant decline in transepidermal water loss was found in Test 1.

The present invention can be practiced in other various forms without departing from the spirit and principal features thereof. In view of this, the embodiments or examples described above merely serve as exemplification in every respect and should not be construed restrictively. A scope of the present invention is defined by claims, and is by no means bound by the text of the specification. Furthermore, all modifications and alternations belonging to the equivalent scope of the claims fall within the scope of the present invention.

## Claims

1. A skin-care water, comprising water from which polyvalent cations are removed and to which sodium ions are added.

2. The skin-care water according to claim 1, which is a moisturizing agent for skin.

3. The skin-care water according to claim 1, which is an improving agent for dry skin.

4. The skin-care water according to claim 1, which is an improving agent for itchy feeling of skin.

5. The skin-care water according to claim 1, which is an improving agent for skin scale.

6. The skin-care water according to claim 1, which is an improving agent for atopic dermatitis.
